# EUROPEAN PATENT SPECIFICATION

(11) **EP 2 201 926 B1**
(45) Date of publication and mention of the grant of the patent: **14.09.2011**
(21) Application number: 08022461.1
(22) Date of filing: 24.12.2008
(51) Int. Cl.: A61K 8/06, A61K 8/26, A61K 8/34, A61K 8/92, A61Q 15/00, A45D 34/04

(54) **Antiperspirant compositions and products**
Schweißhemmende Zusammensetzungen und Produkte
Compositions anti-transpirantes et produits

(43) Date of publication of application: 30.06.2010
(73) Proprietor: Unilever PLC, A Company Registered in England and Wales under Company no. 41424, London EC4Y 0DY Greater London (GB); UNILEVER NV, 3013 AL Rotterdam (NL)
(72) Inventor: Archer Margaret, Seacroft, Leeds, LS14 2AR (GB); Butterworth, Andrew, Seacroft, Leeds, LS14 2AR (GB); Ferrier, Lindsay Karen, Seacroft, Leeds, LS14 2AR (GB); Jones, Shirley, Seacroft, Leeds, LS14 2AR (GB); Brennan, Gail Christine, Bebbington, Wirral, Merseyside, CH63 3JW (GB); Williams, Jason Richard, Bebbington, Wirral, Merseyside, CH63 3JW (GB); Polonka, Jack, Trumbull, CT 06611 (US)
(74) Representative: Whaley, Christopher

(56) References cited:
- US-A1- 2002 155 078
- US-A1- 2003 199 660
- US-A1- 2004 091 439
- US-A1- 2008 279 902

## Description

The present invention relates to antiperspirant compositions and more particularly to aqueous compositions that are applicable using a roll-on dispenser, and to products in which an antiperspirant emulsion is contained within a dispensing roll-on container.

### Background and Prior Art

For many years, humans have employed cosmetic methods, sometimes alternative referred to as non-therapeutic methods, to prevent or at least ameliorate bodily functions which society at the time under consideration considers to be unsightly or otherwise undesirable. These methods have included controlling the appearance of sweat by topical application of an active which prevents egress of sweat from the eccrine glands. The active can be applied cosmetically and topically to the skin, broadly speaking, by one of two methods. Different consumers prefer one method or the other. In one method, sometimes called a contact method, a composition is wiped across the surface of the skin, depositing a fraction of the composition as it passes. In the second method, sometimes called the non-contact method, the composition is sprayed from a dispenser held proximate to the skin, often in the region of 10 to 20cms. The spray can be developed by mechanical means of generating pressure on the contents of the dispenser, such as a pump or a squeezable sidewall or by internally generated pressure arising from a fraction of a liquefied propellant volatilising, the dispenser commonly being called an aerosol.

Liquid compositions that are applicable from a roll-on broadly speaking can be divided into two classes, namely those in which a particulate antiperspirant active is suspended in a hydrophobic carrier, such as a volatile silicone and those in which the antiperspirant active is dissolved in a carrier liquid. The latter has proven to be more popular. There are mainly two sorts of dissolving carrier liquid, namely carriers that are predominantly alcoholic, which is to say the greater part of the dissolving carrier fluid comprises ethanol and the second class in which the carrier liquid is mainly water. The former was very popular because ethanol is a mild bactericide in its own right, but its popularity waned because it stings, especially if the surface onto which the composition has been applied has been damaged or cut, such as can easily arise during shaving or other de-hairing operations.

The second class of formulations that is an alternative to alcoholic formulations comprise a dispersion of water-insoluble or very poorly water soluble ingredients in an aqueous solution of the antiperspirant. Herein, such compositions will be called emulsions. Antiperspirant roll-on emulsions commonly comprise one or more emulsifiers to maintain a distribution of the water-soluble ingredients.

In addition to offering excellent control (inhibition) of sweating, consumers are now seeking additional benefits. Many consumers would like their armpits to be attractive and/or comfortable as well as dry, or at least be less unattractive and/or less uncomfortable than when employing 20^{th} century formulations. In particular, consumers are looking for products that offer an improved tone and/or radiance and/or an improved smoothness in appearance. Perception of such improved attributes can offer confidence to the user and thereby promote the well being and happiness of the user.

Emulsion Roll-on formulations have been developed that can improve the moisture retention of skin to counteract the effect of employing the antiperspirant and thereby improve the elasticity of the skin, for example by incorporating glycerol or/and a low molecular weight polyethylene glycol (eg 4 to 10 units), but such formulations do not substantially alter the perception of the skin by the user in terms of tone, radiance or smoothness.

US 2002/155078 A1 (AVENDANO ESTHER [MX] ET AL) 24 October 2002 (2002-10-24) discloses (ex. 3) an antiperspirant emulsion, suitable for roll -on use (Claim 1), comprising:
- an aqueous phase in which is dissolved an antiperspirant salt: Al Zr tetrachlorohydrex Gly
- at least 1 % by weight of a water -soluble aliphatic di or tri -hydric humectant: PG
- at least 1.5% by weight of a nonionic emulsifier: Structure Solanace
- at least 0.003% by weight of a mica: Timiron.

### Object of the present invention

It is an object of at least some embodiments of the present invention to devise antiperspirant compositions that improve the skin appearance of skin to which an emulsion roll-on formulation has been applied, or at least the perception of such attributes by the user.

It is a further object of at least certain embodiments of the instant invention to devise an antiperspirant composition in the form of an emulsion that improves the perceived appearance of skin.

It is a yet further object of various embodiments of the instant invention to devise an antiperspirant product that enables an antiperspirant emulsion when applied to skin to have a perceived improved appearance.

Other and further objects may become apparent in the subsequent text herein.

### Brief summary of the present invention

According to one aspect of the present invention, there is provided a cosmetic antiperspirant roll-on emulsion comprising
an aqueous phase in which is dissolved an antiperspirant salt;
at least 1% by weight of a water-soluble aliphatic di or tri-hydric humectant or/and a glyceride oil;
at least 0.25% by weight of a fragrance oil;
at least 1.5% by weight of a nonionic emulsifier and
at least 0.25% by weight of a dispersed mica pigment.

By dispersing the mica pigment within the composition and in the presence of the humectant and/or the glyceride oil, the composition enjoys at least one of increased lustre, brightness and smoothness. This manifests itself as improved perceived tone and/or radiance

According to a second aspect of the present invention there is provided a means for improving the perceived appearance of skin to which an aqueous antiperspirant emulsion has been applied by distributing within the composition at least 0.25% by weight of a mica pigment.

According to a third aspect of the present invention, there is provided an antiperspirant product which comprises an aqueous antiperspirant emulsion containing at least .25% of a mica pigment contained within a dispensing container comprising a reservoir defining at one a retaining housing for a ball that partially protrudes outside the housing, which housing is further provided with one or more or means for perturbing fluid flow outward of the ball within the housing and/or means for controlling the film of fluid adhering to the ball on its rotation.

By controlling the flow of fluid, which in this context includes both liquid and gas, and/or the film of fluid on the ball in conjunction with a mica-pigment-containing composition, the composition can be applied particularly effectively.

In a further aspect of the instant invention there is provided a process for improving the perceived appearance of skin to which an antiperspirant composition has been applied comprising the step of bringing the ball of the product of the third aspect into contact with and rolling it across the surface of the skin, especially in the axilla.

### Detailed description of the present invention, including preferred embodiments.

The present invention relates to means for improving the visual appearance and particularly the perceived appearance, of skin to which an aqueous antiperspirant emulsion has been applied.

### Glyceride oil

In a number of highly desirable embodiments, the invention compositions contain a glyceride oil and particularly a natural glyceride oils. In preferred glyceride oil, glycerol is esterified with one or more olefinically unsaturated C₁₈ fatty acids.. In many instances, the oils comprise one or more triglycerides. The fatty acid residues in the oils can comprise, commonly, from one to three olefinic unsaturated bonds and often one or two. Whilst in many instances the olefinic bonds adopt the trans configuration, in a number of desirable products the bond or bonds adopt the cis configuration. If two or three olefinic unsaturated bonds are present, they can be conjugated. The fatty acid can also be substituted by an hydroxyl group. The natural oils employable herein desirably comprise one or more triglycerides of oleic acid, linoleic acid, linolenic acid or ricinoleic acid. Various isomers of such acids often have common names, including linolenelaidic acid, trans 7-octadecenoic acid, parinaric acid, pinolenic acid punicic acid, petroselenic acid and stearidonic acid. It is especially desirable to employ glycerides derived from oleic acid, linoleic acid or petroselenic acid, or a mixture containing one or more of them.

Natural oils containing one or more of such triglycerides include coriander seed oil for derivatives of petroselinic acid, impatiens balsimina seed oil, parinarium laurinarium kernel fat or sabastiana brasilinensis seed oil for derivatives of cis-parinaric acid, dehydrated castor seed oil, for derivatives of conjugated linoleic acids, borage seed oil and evening primrose oil for derivatives of linoleic and linolenic acids, aquilegia vulgaris oil for columbinic acid and sunflower oil, olive oil or safflower oil for derivatives of oleic acid, often together with linoleic acids.

Other suitable oils are obtainable from hemp, which can be processed to derive stearadonic acid derivatives and maize corn oil. An especially convenient natural oil by virtue of its characteristics and availability comprises sunflower oil, ranging from those rich in oleic acid glycerides to those rich in linoleic acid glycerides, rich indicating that its content is higher than that of the other named acid.

The proportion of the natural oil such as triglyceride oil in the composition is often selected in the range of from 0.1 to 10% by weight of the carrier mixture, especially in the range of from at least 1% by weight and particularly at least 0.5%. Often, its weight proportion is selected in the range of up to 6% by weight and in many embodiments up to 4% of the carrier oils. A particularly convenient range comprises from 0.75 to 3% w/w of the carrier oils.

### Humectant

The compositions according to the present invention preferably comprise a di or trihydric humectant. By including such a material, the demoisturising effect of an astringent antiperspirant salt can be counteracted to at least some extent. Accordingly, the humectant cooperates with the other ingredients, such as in particular, the mica pigment and if present the triglyceride oil and, again if present, any occlusive oil to provide not only a short term improvement in visual appearance of the skin to which the composition is applied, compared with applying compositions from which such beneficial ingredients are absent, but by prolonged use over for example weeks or months can improve the inherent quality of the skin, reducing the likelihood of visible imperfections and dry patches that can be instantly addressed by the incorporation of a smoothing aid into the antiperspirant composition.

The humectants contemplated herein are suitably propylene glycol, and preferably glycerol and/or polyethylene glycol (PEG) having a molecular weight of from 200 to 600, such as from 250 to 500.

The weight proportion of the selected humectant, or mixture of humectants, is desirably at least 1%, and preferably at least 2.5%. Its proportion is attractively not more than 10% and in many desirable embodiments is up to 7.5% by weight of the composition.

An essential constituent of the invention compositions is a mica pigment. Such pigments are obtained by applying a thin coating of titanium dioxide, optionally with tin oxide and/or possible silicon dioxide to the surface of the mica, which often have adopted the physical form of platelets. In some instances, the coating further incorporates a minor fraction of a transition metal oxide, including in particular iron, chromium, copper or cobalt oxides or a combination of two or more of them. By incorporating the metal oxide, the resultant material exhibits a colour highlight that supplements the reflective character of the substrate mica. Such pigments are commonly called interference pigments. It is particularly to select an interference pigment that exhibits a highlight having a wavelength of below 550nm, particularly below 500nm. Many preferred pigments exhibit a highlight of wavelength greater than 400nm and particularly from 450nm. In other words, interference pigments having a violet or indigo tint are preferred and those having a bluish tint are much preferred. Suitable and/or preferred mica pigments including mica interference pigments are commercially available, such as various grades from Merck Inc under their trade mark Timiron.

The invention compositions incorporate at least 0.25% by weight of the mica pigment that is distributed through the composition. Advantageously, in total, at least 0.5% by weight mica pigment and/or interference pigment is incorporated, and especially at least 0.6% by weight. Its weight proportion is often up to 2.5% and in many suitable embodiments is up to 1.2%.

Highly desirably, the mica pigment is present in a weight ratio to the humectant of from 2:5 to 1:10 and particularly from 1:4 to 2:15, such as 1:4 to 1:6.

Very desirably, the mica pigment is present in a weight ratio to the glyceride oil of from 2:5 to 1:10 and particularly from 1:4 to 2:15, such as 1:4 to 1:6.

Herein, one or a mixture of mica pigments can be employed. One especially desirably combination comprises a mixture of pigments of which one comprises a blue tint and a second a silver tint, present in a weight ratio at he discretion of the producer, and often in the range of from 5:2 to 2:5. In particularly desirable compositions the weight ratio is from 5:4 to 4:5, and especially 1:1.

The mica pigment desirably comprises at least 95% by weight of its particles below 60µm. In many attractive embodiments, the mean particle size (D-50) of the mica pigment, or at least one of them in a mixture, is from 12 to 25µm and advantageously the mean particle size (D-50) of the mixture is in the range of from 12 to 25µm, and especially form 12 to 17µm. It is especially desirable that at least one of the mica pigments has a mean particle size (D-50) of below 15 µm, and especially a pigment having a violet or particularly a bluish tint.

The interference pigment cooperates with humectant and/or the glyceride oil to enhance the visible appearance of the skin to which the composition has been topically applied.

In a further aspect of the instant invention there is provided an antiperspirant emulsion comprising an antiperspirant salt dissolved in an aqueous phase, an emulsifier and an oil, which may be a fragrance oil, further containing the mica pigment in an amount of at least 0.25% by weight that exhibits a highlight having a wavelength of above 400nm and below 500nm and preferably greater than 400nm, optionally together with a further mica pigment. Preferences in regard to the mica pigment and mixture of mica pigments are shown herein above, including the total amount of pigment, the particle size of the pigments and the presence of one or more additional ingredients in the emulsion.

In a yet further aspect of the instant invention there is provided an antiperspirant emulsion comprising an antiperspirant salt dissolved in an aqueous phase, an emulsifier and an oil, which may be a fragrance oil, further containing the mica pigment in an amount of at least 0.25% by weight that has a mean particle size (D-50) of from 5 to 15µm, optionally together with a further mica pigment, the mean particle size (d-50) of the mixture of mica pigments being up to 20µm and preferably between 10 and 17µm. Preferences in regard to the mica pigment highlight and mixture of mica pigments are shown herein above, including the total amount of pigment, the particle size of the pigments and the presence of one or more additional ingredients in the emulsion.

A particular constituent of the antiperspirant compositions herein is the antiperspirant active itself. The weight proportion of the astringent antiperspirant salt, in the composition or mixture if more than one salt is employed, is varied at the discretion of the manufacturer and normally in the range of from 5 to 30%, and in many desirable compositions at least 10 or 15%, such as up to 20 or 26% by weight.

Astringent salts employed herein are often selected from astringent aluminium, zirconium and mixed aluminium/zirconium salts, optionally complexed. Preferred aluminium, zirconium and aluminium/zirconium salts contain a halide, especially chloride and especially preferred salts are basic salts, which is to say a fraction of the halide within the empirical formula has been replaced by bound hydroxyl groups, such as at least half. Chlorohydrate salts are very highly desired.

Aluminium halohydrates are usually defined by the general formula Al₂(OH)ₓQ_{y}.wH₂O in which Q represents chlorine, bromine or iodine, x is variable from 2 to 5 and x + y = 6 while wH₂O represents a variable amount of hydration. Aluminium chlorohydrate as made comprises a mixture of a number of different polymeric species in varying proportions, depending on the molar ratio of aluminium to chloride and the conditions employed during manufacture. All such mixtures are employable herein. Commonly called activated aluminium chlorohydrate or enhanced activity aluminium chlorohydrate, sometimes abbreviated to AACH, can be contemplated, in which the proportion of the more active species, such as Band III species (by a conventional chromatographic method) is higher by virtue of its method of manufacture. In one definition of activated, given in EP 6739, the material has greater than 20% Band III.

Zirconium actives can usually be represented by the empirical general formula: ZrO(OH)_{2n-nz}B_{z}.wH₂0 in which z is a variable in the range of from 0.9 to 2.0 so that the value 2n-nz is zero or positive, n is the valency of B, and B is selected from the group consisting of chloride, other halide, sulphamate, sulphate and mixtures thereof. Possible hydration to a variable extent is represented by wH₂0. Preferable is that B represents chloride and the variable z lies in the range from 1.5 to 1.87. In practice, such zirconium salts are usually not employed by themselves, but as a component of a combined aluminium and zirconium-based antiperspirant.

The above aluminium and zirconium salts may have coordinated and/or bound water in various quantities and/or may be present as polymeric species, mixtures or complexes. In particular, zirconium hydroxy salts often represent a range of salts having various amounts of the hydroxy group. Zirconium aluminium chlorohydrate may be particularly preferred.

Antiperspirant complexes based on the above-mentioned astringent aluminium and/or zirconium salts can be employed. The complex often employs a compound with a carboxylate group, and advantageously this is an amino acid. Examples of suitable amino acids include dl-tryptophan, dl-β-phenylalanine, dl-valine, dl-methionine and β-alanine, and preferably glycine which has the formula CH₂(NH₂)COOH.

It is highly desirable in some embodiments of the instant invention in which emphasis is -laced upon antiperspirant efficacy to employ complexes of a combination of aluminium halohydrates (especially chlorohydrates) and zirconium chlorohydrates together with amino acids such as glycine, which are disclosed in US-A-3792068 (Luedders et al). Certain of those Al/Zr complexes are commonly called ZAG in the literature. ZAG actives generally contain aluminium, zirconium and chloride with an Al/Zr ratio in a range from 2 to 10, especially 2 to 6, an Al/Cl ratio from 2.1 to 0.9 and a variable amount of glycine. Actives of this preferred type are available from Westwood, from Summit and from Reheis.

In other embodiments, in which the emphasis is placed on composition mildness, it is preferred to aluminium chlorohydrate that is at least substantially free from zirconium, by which is meant that the mole ratio of Al:Zr is >20:1 and especially >50:1, and especially is totally free.

It is particularly preferred for the antiperspirant salts to be at least substantially free from aluminium sulphate, by which is meant that its weight proportion of the total weight of all antiperspirant salts present is less than 5%, especially less than 3% and particularly less than 1%. Total absence would be very suitable.

The antiperspirant salt is dissolved in the aqueous phase.

An essential constituent of compositions of the present invention is a non-ionic emulsifier or mixture of emulsifiers forming an emulsifier system. Such an emulsifier system conveniently has a mean HLB value in the region of from about 5 to about 12 and particularly from 6 to about 10. An especially desired mean HLB value is from 7m to 9. Such a mean HLB value can be provided by selecting an emulsifier having such an HLB value, or more preferably by employing a combination of at least two emulsifiers, a first (lower) HLB emulsifier having an HLB value in the range of from 2 to 6.5, such as in particular from 4 to 6 and a second (higher) HLB emulsifier having an HLB value in the range of from about 6.5 to 18 and especially from about 12 to about 18. When a combination of emulsifiers is employed, the average HLB value can be obtained by a weight average of the HLB values of the constituent emulsifiers.

An especially desirable range of emulsifiers comprise a hydrophilic moiety provided by a polyalkylene oxide (polyglycol), and a hydrophobic moiety provided by an aliphatic hydrocarbon, preferably containing at least 10 carbons and commonly linear. The hydrophobic and hydrophilic moieties can be linked via an ester or ether linkage, possibly via an intermediate polyol such as glycerol.

Preferably the hydrophobic aliphatic substituent contains at least 12 carbons, and is derivable from lauryl, palmityl, cetyl, stearyl, olearyl and behenyl alcohol, and especially cetyl, stearyl or a mixture of cetyl and stearyl alcohols or from the corresponding carboxylic acids. It is particularly convenient to employ an emulsifier comprising a polyalkylene oxide ether.

The polyalkylene oxide is often selected from polyethylene oxide and polypropylene oxide or a copolymer of ethylene oxide and comprises a polyethylene oxide. The number of alkylene oxide and especially of ethoxylate units within suitable emulsifiers is often selected within the range of from 2 to 100. Emulsifiers with a mean number of ethoxylate units in the region of 2 can provide a lower HLB value of below 6.5 and those having at least 4 such units a higher HLB value of above 6.5 and especially those containing at least 10 ethoxylate units. A preferred combination comprises a mixture of an ethoxylate containing 2 units and one containing from 10 to 40 units. Particularly conveniently, the combination of emulsifiers comprises steareth-2 and a selection from steareth-15 to steareth-30.

It is desirable to employ a mixture of ethoxylated alcohol emulsifiers in a weight ratio of emulsifier having a lower HLB value of <6.5 to emulsifier having a higher HLB value of >8 of from 1.5:1 to 6:1 and particularly from 2:1 to 5:1.

The total proportion of emulsifiers in the composition is usually at least 1.5% and particularly at least 2% by weight. Commonly the emulsifiers are not present at above 6%, often not more than 5% by weight and in many preferred embodiments up to 4% by weight. An especially desirable concentration range for the emulsifiers is from 2.5 to 4% by weight.

An optional constituent of the composition comprises a particulate silica such as an amorphous silica, eg a fumed silica, preferably a fumed (sometimes called pyrogenic) silica which has been hydrophobically treated. Such materials are commercially available under the name hydrophobic silica. Hydrophobic silicas are obtained by chemically bonding a hydrophobic substituent such as especially a siloxane group onto the surface of the silica, possibly following an intermediate treatment in which the surface of the silica has been rendered hydrophilic. Suitable reactants to generate a hydrophobic substituent include halosilanes and in particular chlorosilanes and methylated silazanes such as hexamethyldisilazane. It is particularly desirable to employ a silica that is capable of thickening an oil such as a plant oil.

Desirably, the silica, such as the fumed silica, and especially the hydrophobic silica has a BET specific surface area of at least 100 m²/g and particularly from 150 to 400 m²/g. The silica comprises very fine particles, fumed silica commonly having a diameter for individual particles of below 40 nm and in many instances at least 99% by weight of below 40 nm. In fumed silica as supplied, some aggregation can occur so that in many embodiments, the supplied silica has an average particle size (diameter) of less than or equal to 1000 nm, preferably less than or equal to 500 nm, i.e. the diameter of the silica particle of average weight. In at least some desirable embodiments, at least 99% by weight of the silica particles, as supplied, are in the range of 10 to 500 nm..

The weight proportion of silica in the formulation is often selected taking into account the desired viscosity of the eventual formulation, together with other attributes such as its effect on the speed of drying of the formulation, its perceived greasiness and/or its perceived stickiness. The weight concentration of silica in some embodiments of the composition is desirably at least 0.2%, often at least 0.3% and in many desirable embodiments is at least 0.5% by weight. Its concentration is commonly not greater than 2%, often not greater than 1.5% and in a number of very desirable formulations is not higher than 1.0%. A preferred weight range of silica concentrations is from 0.6 to 0.8%.

The water content of the composition is commonly selected in the range of from 50 to 93% by weight and often from 60 to 85% by weight.

The compositions herein comprise a perfume (fragrance) oil, which is normally present at a concentration of up to 4% and in many formulations from 0.25 to 2% or 2.5% by weight of the emulsion. The composition can contain as perfume, at the discretion of the producer, free fragrance, a profragrance, encapsulated fragrance or fragrance that is associated with a host substrate such as cyclodextrin, or a mixture of any two or more of such perfume options. The term fragrance includes oils that mask any malodorous ingredient.

In addition to the glyceride oil and perfume oil, the composition can comprise, if desired, a volatile oil, and in particular a volatile silicone oil. The weight proportion of such an oil is often selected in the range of up to 20% by weight, such as at least 10%, for example up to 17.5% by weight of the emulsion. The volatile silicone oil conveniently can be selected from dimethicones and cyclodimethones oils containing up to 6 silicon atoms, especially 4, 5 or 6 silicon atoms and particularly a blend of oils comprising at least 95% by weight compounds with 5 or 6 silicone atoms. Suitable volatile silicone oils are available commercially from Dow Corning and General Electric.

The silicone oil can serve as a vehicle to introduce a silicone elastomer, by which is meant a crosslinked dimethicone. The elastomer is typically crosslinked by reacting a silicone hydride with an α-ω olefinically unsaturated dialkylene. The elastomer is conveniently incorporated in the emulsion at a concentration of at least 0.1 % up to 3%, and especially from 0.5% to 2% by weight of the antiperspirant emulsion. Elastomers are commercially available, for example from Dow Corning Inc and Shinetsu

In some especially desirable embodiments, the invention compositions comprise in addition to the mica pigment, a hair growth inhibitor. Advantageously, the hair growth inhibitor is present at a concentration of at least 0.001 % and often up to 0.01 %. The weight ratio of mica pigment to hair growth inhibitor is often selected in the range of from 200:1 to 1000:1, and particularly from 400:1 to 800:1. By employing a hair growth inhibitor in conjunction with the mica material, the composition not only improves the appearance of the skin on application, but the effect is maintained and increased with consecutive applications of the composition. The hair growth inhibitor can be oil-soluble, such as palmatine and be included in a disperse oil phase, such as dissolved in the glyceride oil and optional volatile silicone oil. It is particularly suitable to employ a water-soluble hair growth inhibitor or a combination of water-soluble materials that together perform that function. The mixture of water-soluble ingredients advantageously comprises soy protein or a hydrolysed soy protein, and desirable further comprises one or more extracts from one or more of hypericum perforatum (St John's wart), hamamelis virginiana (witch hazel), arnica Montana flower and salix alba (willow) bark extract, and/or urea.

For example, reduced hair growth reduces the need for shaving or plucking and therefore the skin is challenged less frequently, consequently with less redness or blotchiness arising that needs combating by the mica. The combination therefore generates healthier-looking skin than either constituent alone.

A further desirable constituent comprises a micro-fine aluminium oxide powder, such as Spectra-Al in a concentration of up to 5% by weight, and in particular from 1.5 to 3.5% by weight. Incorporation of such a particulate material together with the mica pigment can achieve a noticeable improvement in one or more of perceived tone, radiance and smoothness of axilla skin, and particularly when employed together with a mica pigment exhibiting a silver tint, for example one having a mean particle size (D-50) of fro 18 to 25µm. Advantageously, the weight ratio of aluminium oxide powder to mica pigment can be selected in the range of from 2:1 to 6:1 and a combined weight in the range of from 1.2 to 5%.

If desired, the composition can comprise a supplementary deodorant active, i.e. an active other than the antiperspirant salt. Suitable supplementary deodorant actives can comprise deodorant effective concentrations of deoperfumes, and/or microbicides, including particularly bactericides, such as chlorinated aromatics, including biguanide derivatives, of which materials known as Igasan DP300™ (triclosan), Tricloban™, and Chlorhexidine warrant specific mention. A yet another class comprises biguanide salts such as are available under the trade mark Cosmocil™. Supplementary deodorant actives are commonly employed at a concentration of from 0.1 to 5% by weight and often up to 1% by weight of the composition.

### Method of Manufacture

The compositions according to the present invention can be made conveniently in accordance with processes that have been employed hitherto using the same ingredients in the absence of the mica pigment employed herein to make antiperspirant emulsion compositions.

Preferably, the emulsion is made by first preparing separate aqueous and oil mixtures which are brought together before shearing. The aqueous phase commonly contains the antiperspirant active. Where a mixed emulsifier system is employed, it is often desirable to incorporate any emulsifier having a low HLB value, particularly of <6.5 into the oil phase and an emulsifier having a high HLB value, particularly of >6.5 into the aqueous phase. The temperature of the respective phases can be raised, where necessary, to accelerate dissolution of the emulsifier, for example to above 50°C.

It is often convenient to incorporate particulate materials such as the mica pigment, and aluminium powder and/or silica, if employed, in the aqueous phase.

It is preferable to incorporate the fragrance oil last of all and shortly before the entire mixture is sheared, especially when either or both phases have been heated so as to accelerate emulsifier dissolution.

### Dispensers

The compositions produced herein are suitable for dispensing from known cosmetic roll-on dispensers. Such dispensers comprise a reservoir for the antiperspirant emulsion defining at one end a housing for retaining a roll-ball having a segment that protrudes outside the housing, the one end typically being covered by a removable cap. A suitable example of such a dispenser is described in EP1175165 and a suitable invert dispenser is described in USP6511243.

In some preferred embodiments of the instant invention, and in the third aspect of the instant invention, the antiperspirant emulsion containing at least 0.25% by weight of the mica pigment is contained in a roll-on dispenser for a liquid comprising a bottle and a removable cap, the bottle having an interior and a first end which defines a housing for a rotatable spherical ball, said housing having a chamber within which the ball can rotate having an inward end in fluid communication with the interior of the bottle and comprising a lateral sealing ring dimensioned to prevent the ball being urged into the interior of the bottle when subject to inward axial force, an opposed outward end dimensioned to retain the ball and a side-wall having an interior surface extending between the outward end and the inward end the ball having a segment projecting outside the housing, the cap having means to urge the ball axially towards the sealing ring, in which at least one fluid flow purturbator is located on the interior surface of the chamber in or outward of the sealing ring or/and means to control the depth of liquid film adhering to the ball during rotation.

Herein the term "inward" when employed axially, for example in "inward end" in respect of a housing intended for mounting on or an integral mounding with a bottle reservoir refer respectively to the end adjacent to the interior of the bottle, and outward is that remote from the interior. Axial relates to an axis extending centrally through the inward and outward ends of the housing.

Herein, the terms upward, downward, above and below when employed in respect of the dispenser and its constituent parts refer to when the dispenser is in an upright orientation, which is to say the cap is above the bottle.

In such preferred embodiments, the fluid flow within the housing is modified by incorporating at least one perturbator within the housing chamber, including, in particular, modifications to the interior surface of the housing for the ball which result in localised disruption of or modification the flow of fluids across that surface.

The interior surface of the housing chamber can be modified in a number of different ways to perturb fluid flow over the surface. This can comprise the introduction of baffles, preferably baffles on the interior face which can possibly introduce turbulence into the fluid flow. Conveniently, the baffles can comprise one or more continuous or discontinuous shallow lateral beads intermediate between the outward end of the housing and the lateral sealing ring. If desired, the beads can also be castellated or have an axial component, as for example the beads being arranged in chevrons or an array of short sections distributed symmetrically or randomly across the interior face of the housing. Shallow indicates that the bead does not span the gap between the housing wall and the rotating ball. The beads commonly have a depth of from about 50 to 500 µm, so as to achieve fluid perturbation but not obstruct the outward of flow of liquid from the dispenser excessively. Where lateral beads are employed, such as two beads, their axial spacing from each other is often selected in the range of from 2 to 8 mm such as from 2.5 to 4.5 mm. The bead or closest bead is often in the range of from 2 to 8 mm axially outward from the sealing ring and particularly from 2.5 to 4.5 mm. The bead or closest bead is often from 5 to 10 mm axially inward of the outward end of the housing.

A further and especially desirable way in which the ball housing surface can be modified to cause fluid perturbation in the chamber comprises notching the lateral sealing ring with a multiplicity of axially extending notches at its upward edge i.e. the edge pointing towards the outward end of the housing. The notches are preferably made all around the sealing ring, and especially they are approximately equidistant from their neighbours. The number of notches is at the discretion of the manufacturer, but preferably selected to provide a spacing in the range of from 2.5 to 7.5 mm and particularly in the range of from 3 to 5 mm for hand-held cosmetic dispensers employing a roll-ball of from 25 to 35 mm diameter such as is particularly suitable for dosing cosmetic products such as deodorants and antiperspirants. For a sealing ring having a circumference of from 90 to 110 mm, this indicates the number of notches conveniently being in the range of from 15 to 36, such as particularly from 20 to 30.

The notches are dimensioned and profiled, in practice, so as to encourage fluid turbulence. The width of each notch is often from about 250 or 500 to 2000 µm, such as from 750 to 1500 µm. The axial depth of each notch is often at least 500 µm and commonly not greater than 2500 µm, and in a number of preferred embodiments is from 1200 to 1600 µm. The notches advantageously have a sharp outward edge, such as from about 85 to 110°, and preferably square edged. The notches themselves are commonly rectangular in cross section, possibly having tapering sides.

Advantageously, the notches on the sealing ring are in fluid connection with an annular, preferably v- or u-shaped groove formed in the side-wall of the housing beside the upward-facing edge of the sealing ring and. This lateral v- or u-shaped groove conveniently has a width (at its upward end, i.e. its mouth) and axial depth which are each desirably selected in the range of from 500 to 2500. Its depth is advantageously similar to the depth of the notches in the sealing ring, and preferably is the same.

Advantageously, the housing employs both the baffles and the notched sealing ring.

The instant invention may in at least some embodiments be considered to comprising the provision of a housing for a roll-on which comprises one or more baffles such as a bead or beads as described herein and/or, especially, the notching of the outward facing edge of a sealing ring on the interior face of the housing as described herein, particularly when it is in communication with an annular groove, irrespective of the reason why they smooth variations in the dose of a cosmetic liquid applied by the dispenser.

The housing can additionally have, mounted therein, a spider positioned between the sealing ring and the interior of the bottle. Such a spider can comprise a plurality of spokes, optionally with an intermediate ring linking two or more of the spokes. The spokes can be fixed, that is to say have both ends secured, for example one end being mounted on the interior face of the housing and the other end fixed to a hub, or can be free, which is to say has one end fixed, for example being mounted to the housing wall or to the aforementioned hub. It is particularly desirable to employ a spider having a convex profile proximate to the ball. Use of such a particularly desirable spider, that can wipe excess liquid off the ball to leave a film of pre-determined depth, can contribute to the reduction of fluctuations between successive topical applications of the cosmetic formulation.

A preferred spider herein comprises a plurality of spokes which can as a minimum be two, provided that together they subtend a wide arc of the housing, such as preferably at least 120 through to 240 degrees and preferably are at or close to 180 degrees apart. Preferably, the number of spokes is at least 3. and in some instances, the number of spokes is at least 4. The number of spokes is normally no greater than 12, in so as not to constrict the passage of liquid between the spokes unduly, and in several preferred embodiments is not more than 9. A convenient number is 3, 4, 5 or 6 spokes, and especially 6 spokes. Although the spokes can be arranged asymmetrically around the interior of the housing side-wall, it is preferable to employ a symmetrical arrangement, for example point or mirror symmetry.

The spider can be mounted on the interior of the housing side-wall one or more mounting points. When a single mounting point is employed, the spoke leading away from the mounting point terminates at its opposed end in a hub from which radiates at least one further spoke, and preferably from 2 to 5 further spokes. Preferably the spider is mounted on the side-wall at two or more mounting points that themselves are preferably symmetrically arranged around the interior side-wall and conveniently by 3 or 4 mounting points. The mounting points are most desirably equidistant around the housing side-wall and lateral relative to each other, i.e. all at the same axial distance below the widest diameter of the housing.

In many embodiments, the spider comprises a hub from which spokes radiate towards the housing side-wall. If desired, all the spokes can extend between the side-wall and the hub, and for convenience herein these can be called fixed spokes. However, some of the spokes that radiate from the hub and spokes that radiate towards the centre of the housing from the side-wall can have a free end, by which free end is meant that it is not secured to respectively the side-wall or the hub, and for convenience herein these can be called free spokes. It is preferred to employ a mixture of free and fixed spokes, for example in a ratio of from 1:2 to 2:1, and conveniently at 1:1. The free spokes tend to be more flexible whereas the fixed spokes tend to be more rigid and assist in the production of the combined housing and spider, for example in injection moulding. It is especially desirable for fixed and free spokes to be arranged symmetrically, such as 1 or 2 free spokes interposed between adjacent fixed spokes. By adopting a symmetrical arrangement, the ball can be centred more easily, thereby ensuring best that the spokes control the depth of liquid film more evenly. One especially desirable arrangement comprises an even number of spokes in total being 4, 6 or 8 having alternate fixed and free spokes symmetrically arranged around the side-wall.

The spider desirably has a means for providing localised contact with the ball and especially when it has a concave upward, ball-facing surface. Preferably, the spider, is resilient and flexible, at least in an axial direction, or constituent spokes thereof, and mounted so as to bias the ball upwardly, thereby keeping the spider in localised contact with the ball, when the cap is removed. The downward force exerted on the ball maintains contact between ball and spider when the cap is fitted, flexing downwardly the spider, or at least the spokes carrying the boss or pimple.

The localised contact means desirably comprises a boss or pimple standing proud of the surface of the spider, specifically proud of the surface of the spokes facing the ball. The boss or pimple is desirably of round or rounded lateral cross section. The boss or pimple advantageously has a bevelled or rounded chamfer to its contact edge with the ball, thereby to minimise frictional contact with the ball. The boss or pimple advantageously is hemispherical or a cylinder terminating in a hemisphere. The orthogonal height of the pimple, which controls the depth of the liquid film adhering to the ball, is often, for a hand-held cosmetic dispenser, selected in the range of from 300 to 2000 µm and in many instances from 350 to 750 µm.

The pimple or boss often has a diameter of from 300 to 2500 µm and particularly from 350 to 1000 µm, often tapering or rounded to a point (such as below 25 µm diameter) for contact with the ball. The boss or pimple can be employed on free or fixed spokes and on the hub. Advantageously, at least one pimple or boss per spoke is located at a point that is remote from the point of attachment of the spoke, he free to the hub and the fixed to the housing side-wall as the case may be. Particularly desirably each free spoke has a pimple. Most desirably, the pimples present a symmetrical pattern. If desired any spoke can be provided with a plurality of pimples, such as 2 or 3. Preferably when the housing comprises an annular sealing ring between the ball and the spider mounting point(s), any pimple on a spoke mounted on the wall is equidistant between the wall and centre of the housing or closer to the centre.

Alternatively, for a free spoke, the means for spacing the spoke surface from the ball surface can comprise the tip of the spoke being bent upwardly towards the ball, desirably to provide a similar spacing to that provided by a pimple. In the vicinity of the hub, the localised contact can be provided by an upstanding wall that is either continuous or discontinuous and concentric with the housing side-wall.

Desirably a free spoke extends at least about 40% the radius of the interior of the housing, so as to be relatively flexible. In many embodiments the free spoke extends up to 95% of the housing interior radius and especially from 75 to 95% particularly when it extends from a hub. When it extends from the side-wall, the free spoke particularly extends from 50 to 80% of the housing interior radius.

The spokes desirably have a triangular side profile with a concave top wall preferably matching the radius of the ball with the apex of the triangle at the hub, or closest to the hub for a free spoke that is mounted on the side-wall. Such a profile assists the spoke to flex whilst strengthening it adjacent to its mounting point. The spokes can desirably comprise an upstanding wall, often tapered and a base plate, providing a T-shaped cross section. Transversely, the spoke top can be flat or concave, such as with a radius of curvature similar to that of the ball. Advantageously, the spoke sides can be approximately orthogonal to the ball tangent.

Preferably, the concavity of the ball-facing top surface of the spokes has a similar radius to that of the ball so as to enable the film depth to be substantially the same along the length of the spokes. The mounting point of the spokes on the side-wall is such that the ball is radially spaced from the mounting point at the same height as the pimple or like gap-forming means.

The resilient spider often provides a contact force (acting upwardly on the ball) of from 0.01 to 0.1 kg-f and especially in the region of 0.04 to 0.06 kg-f, a force sufficient to maintain contact, but not so great as to render operation of the dispenser difficult.

When a spider mounted inward of the ball is employed, the reservoir and housing are desirable moulded separately and the latter is securely mounted on the former.

The invention is directed in particular to the use of a dispenser employing a ball having a diameter of from about 20 to about 40 mm and especially from about 25 to about 36 mm. Representative ball diameters are 25, 29, 32 or 35.5 mm, or thereabouts.

The housing and spider are preferably injection moulded together in a unitary mould, employing a thermoplastic polymer such as polyethylene or polypropylene.

The compositions of the present invention can be topically applied to skin, and particularly to underarm skin by inverting the dispenser, thereby wetting the ball, if it was stored in an upright orientation, removing the cap, placing the ball in the armpit, and thereafter rolling the dispenser across the skin surface. Commonly the dispenser is rolled at least 4 times back and forth across the skin, at the discretion of the user, depositing commonly at least 0.15 g per arm, such as up to 0.6g.

It is particularly desirable to apply the composition shortly after the armpit has been washed or shaved, and preferably warmed, for example by the application of warm water. The skin at such times is particularly receptive for the application of a smoothing aid, such as the mica pigment to improve the visual appearance of rough skin, of pits, and wrinkles. The composition is thereafter left in place, conventionally, for a period of time commonly between 5 and 24 hours until it is washed off, usually using soap or a conventional shower gel, and water, for example applied using a flannel, loofah or sponge or even fingers. When seeking to inhibit perspiration, the weight of antiperspirant active applied per armpit is often in the range of from 0.15 to 0.5 grams.

Particular embodiments according to the present invention are described hereinafter by way of example only. Such embodiments can be modified by the skilled person in accordance with the foregoing detailed description of the invention.

Comparison CA and Examples 1 to
These Examples and comparison compositions were made by the following general method:-
An aqueous phase was prepared by mixing together in a vessel a 50% aqueous solution of an astringent antiperspirant active, water and any emulsifier having a high HLB value, (>6.5) and heating the mixture until the emulsifier dissolved, typically in the region of 55 to 65°C. The particulate material(s), including mica pigment was incorporated into this aqueous mixture. In a second vessel, an oil phase was prepared by mixing the selected oil with any emulsifier having a low HLB value (< 6.5) and heating the mixture until the emulsifier dissolved, conveniently also in the region of 55 to 65°C. The oil phase was then slowly introduced with continuous stirring into the first vessel. The resultant mixture was allowed to cool to below 40°C and any fragrance was added. The resultant mixture was then passed through a high shear mixer to form an emulsion and charged into roll-on dispensers.

In Comparison CA and Example 1, the dispenser was that described in Figures 1 to 3 of EP1165175. In the remaining Comparisons and Examples, the dispenser was that as illustrated in the appended Figures 1 to 4, in which:-
Figure 1 is a plan view of a housing for a roll ball suitable for mounting on a cosmetic bottle, without the ball in place;
Figure 2 is a cross section view of the housing of Figure 1;
Figure 3 shows a transverse cross section through a free spoke shown in Figure 1;
Figure 4 is a cross section view of a dispenser showing the housing of Figures 1 and 2 with ball in place, mounted on a bottle and having a cap screwed tight.

As illustrated in the Figures, the roll-on dispenser comprised a bottle (1), a spherical ball (2), a housing (3) for the ball (2) integrally moulded with a spider (4) and a cap (5), each of which are moulded from a thermoplastic polymer.

The bottle (1) at its open end has an exterior annular lip (6) and annular groove (7) which snap fit with co-operating annular recess (8) and bead (9) moulding on the inward-face of an annular channel (10) formed by a bifurcated side-wall of the housing (3) dimensioned for a fluid tight fit with the bottle.

The housing (3) comprises an annular side-wall (11) of circular lateral cross section extending between an inward end (12) and an outward end (13). The side wall (11) comprises an upper wall of tapering cross section (14) adjacent to the outward end (13) which has a concave interior face (15) having two lateral shallow beads (20) and screw threads (16) on an exterior face. Shallow annular beads (20) act as baffles which perturb air as it flows into the bottle across the interior face of the housing during use and thereby smooth the application of liquid. The housing (3) has a thickened middle wall section having an inward facing annular sealing ring (17) into the outward (upper) edge of which are cut a multiplicity of short notches (18) equidistantly spaced around the ring extending down about 30% of the axial height of the sealing ring (17), which disrupt the flow of air across the interior face of the housing. The notches (18) are of about the same depth as and in fluid communication with a lateral annular v-shaped groove (19) is defined by an inward face of the upper wall (11) and an outward face of the ring (17), which also provides a small intermediate reservoir for liquid when the dispenser is in an upright orientation. The housing side-wall is bifurcated, providing an inner annular skirt (21) extends into the bottle (1) from the middle section of the housing (3) on which a spider (4) is mounted at three equidistant points (22) around the skirt (21), which spider (4) extends across the inward end (12) of the housing.

The spider (4) comprises three fixed arms (23) extending from the housing skirt (21) to a hub (24) from which radiates three free spokes (25) that each are equidistant from the adjacent fixed spokes (23) and extend about 90% of the distance from the hub to the interior face of the skirt. Each respective spoke (23), (25) has respective concave faces (26) and (27) that face the ball (2) in the housing which has a similar radius of curvature to that of the ball, and is of T-shaped cross section having a strengthening base flanges (28) from which a tapering wall (29) extends upwardly. Each free spoke has at it free end a pimple (30) proud of the concave face (27) which spaces that face (27) from the ball (2) and the pimples (30), being symmetrically arranged, centre the ball. The spokes (23, 25) act as wiper blades regulating the depth of film adhering to the surface of the ball (2) as the ball rotates.

The reservoir (1) has a rounded bottom (5) which prevents the dispenser from standing stably in an upright orientation and a side-wall (6) that has an annular end zone (60) of reduced wall thickness defining a peripheral ledge (7) with an annular groove (8) and an annular bead (9) moulded on its exterior face above the ledge (7).

The housing (2) for the ball (3) comprises an upper side-wall (10) integrally moulded with a middle side-wall (11) and a bifurcated lower side-wall comprising an annular inner wall (12) that is dimensioned to fit within the mouth of the reservoir (1) and an annular outer wall (13) having moulded on its interior face an annular recess (14) and annular bead (15) that engages with corresponding bead (9) and groove (8) on the annular zone (60) of reduced thickness in a snap-fit mounting. A small annular anti-leakage blade (15a) is also integrally moulded on outer wall (13) parallel with and inward of the snap-fit bead (15). The bottom edge of outer wall (13) rests upon ledge (7) in the side-wall (6) when the housing (2) is mounted on the reservoir (1).

The upper side-wall (10) is a truncated hollow hemisphere, defining an outward end of the housing (2) otherwise referred to as a mouth (16) through which ball (3) can be pushed by virtue of its flexibility. The middle wall (11) has a screw thread (21) moulded on its cylindrical exterior face and is of sufficient rigidity to resist deformation during relative rotation of cap (4) to housing (2). The interior face of the middle wall (11) and the inner lower annular wall (12) is approximately truncated hemispherical, having an upward-facing sealing ring (17) in which a multiplicity of square edged short axial notches (18) are cut all the way around its upper edge to a depth of about 30% of the axial height of the sealing ring (17). The ring (17) defines with the middle wall (11) a v-shaped annular groove (19) which can retain fluid when the dispenser is in an upright orientation which has a similar depth to and is in fluid communication with the notches (18). The sealing ring (17) has an upstanding lip (61). The housing interior also has two parallel annular beads (20) between the sealing ring (17) and its mouth (16).

On the interior of the inner annular wall (12) is moulded a resilient flexible spider which consists of three fixed spokes (23), each of which is mounted around the wall (12) at a mounting point (22) at 120 degree intervals and radiating inwardly to intersect at a hub (24) from which radiate outwardly three free spokes (25) equidistantly spaced between adjacent fixed spokes (23). The ball-facing surfaces (26, 27) of the spokes (23, 25) are concave, and have the same radius of curvature as the ball (3) plus the height of a boss (30). The spokes (23, 25) have a transverse inverted T shaped cross section formed by a base flange (28) and an upright wall (29) slightly tapered on both sides, which are nearly parallel to the radius of the ball and hence nearly orthogonal to the tangent of the ball, having ball-facing sharp edges that assist the spokes to act as wipers and transversely having a flat top. A short boss (30) stands proud of the longitudinally extending concave surface (27) at the non-mounted end of each free spoke (25). Together with the exterior surface of the ball (3), the concave surfaces (26, 27) define a passageway (31) controlling the depth of liquid film adhering to the ball (3) as it is rotated, leading top edges of the wall (29) of spokes (23, 25) acting as wiper blades.

The cap (4) has a top wall (32) having a planar exterior which permits the dispenser to stand in an invert orientation, and from its underside a central annular wall (33) depends, of height dimensioned to depress the ball when the cap is attached. The cap (4) has an annular side wall (34) dimensioned to fit over the housing in which is moulded a screw thread (35) adjacent to its mouth and a plurality of axial ribs (36) having a concave contact face (37).

When the cap (4) is fitted by rotation onto the housing (2), screw threads (21 and 35) engage and move the cap (4) axially towards the ball (3), the annular wall (33) and the ribs contact surfaces (37) first contacting the ball (3) and then urging it inwards, i.e. down. As a consequence, the ball (3) is urged into contact with the sealing ring (17), and first of all with its lip (61) and because the ball is always in contact with the bosses (30) on the three free spokes (25), the free spokes are flexed downwardly, especially in the vicinity of the bosses (30) and the spider is distorted.

When the cap (4) is removed, again by rotation, but in the reverse direction, the axial force exerted by the cap is removed and resilient spider returns to its rest position, so that the ball is gently lifted above the sealing ring on bosses (30) and cosmetic fluid can pass between the ball (3) and sealing ring (17) and through a channel (31) of predetermined radial width between the ball and the spider spokes (23, 25). The depth of film adhering to the ball (3) is controlled defined by the height of the bosses (30) on concave surface (27) of the spokes (25). In topical application, when the ball is pressed against the body, such as an armpit, the ball is spaced away from the upper wall section (10) of the housing (2). Flanges (28) at the base of spokes (23, 25) tend to restrict the flow of liquid back into the bottle reservoir when the bottle is turned into an upright orientation.

The following attributes of the compositions were then assessed by the following method and the results summarised in Table 1 below:-
- 10 Female panellists shaved their own axillae approx 12 hrs before the study commenced and their underarms were assessed by an expert clinician in a well lit booth of a clinical test centre.
- Skin quality was scored on a 0 to 5 scale for tone, imperfections, radiance, visual smoothness and visual and the result recorded
- Test product was applied to the axillae by the assessor by wiping the stick 4 times across each axilla in a ventilated booth
- The Expert clinician assessment reassessed the skin quality attributes 10 minutes after product application on the same 0 to 5 scales in the same booth and the results recorded, except in Example 2 in which the assessment was made immediately after application.
- The difference between the score before and after application of the test product for each attribute was calculated and the average is summarised in Table 1 below, increases in tone, radiance and smoothness are recorded as positive as are decreases in imperfections.

Accordingly, the data summarised in Table 1 indicates the effectiveness of the test composition at improving the skin appearance.

The attributes were assessed by mapping onto the following scale (description of severity, coverage of axillae):-

| | |
|---|---|
| 0 | none |
| 1 | light, up to 10% |
| 2 | mild, 11 to 25% |
| 3 | moderate, 26 to 50% |
| 4 | marked, 51 to 75% |
| 5 | severe, 76 to 100% |

The attributes assessed were as follows:-
- Tone: unevenness, freckles, hyperpigmentation through to evenness
- Radiance: dullness through healthy glow to brightness
- Smoothness: visible roughness to velvety, peachy

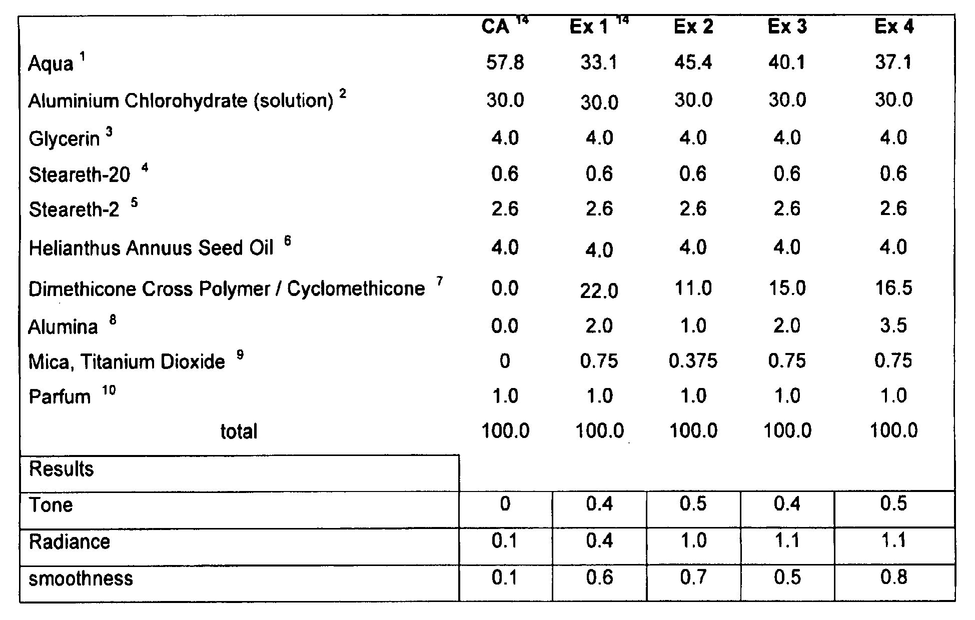

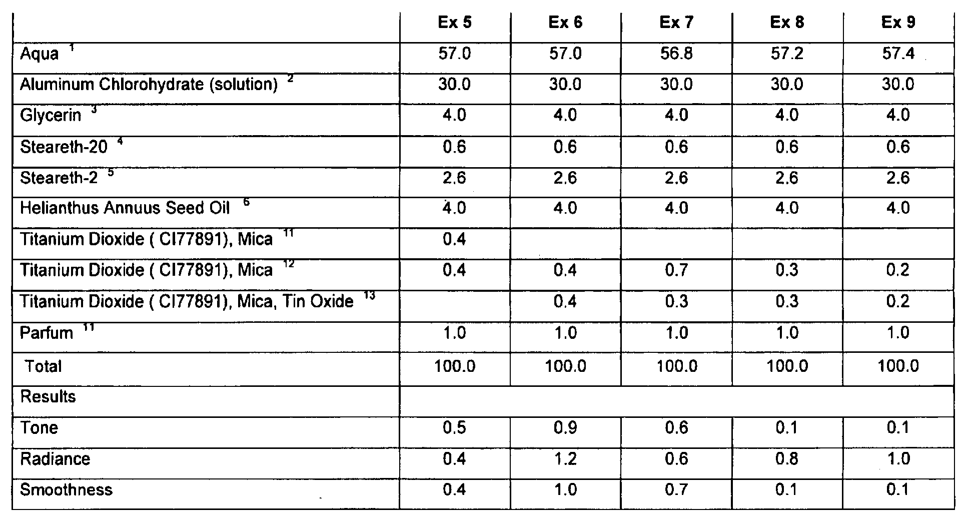

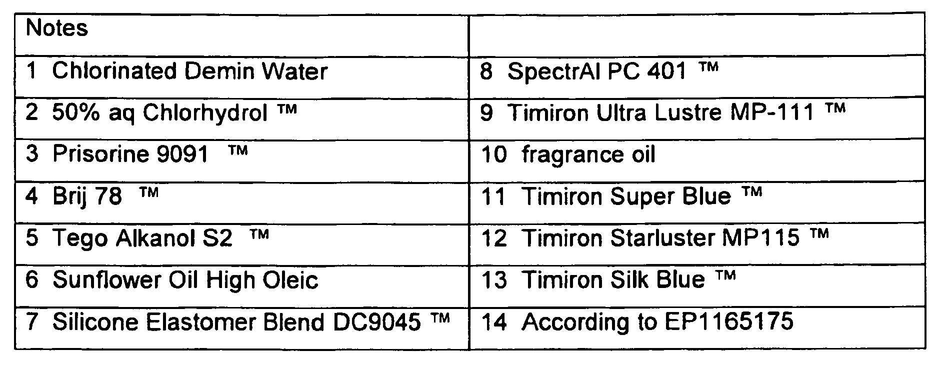

Materials 9, 12, and 13 all comprised >95% by weight of particles between 10 and 60 µm diameter and 14 >95% by weight of particles between 5 and 25 µm diameter. Materials 9, 12 and 13 each had its mean (D-50) particle size of between 18 and 25 µm and material 14 between 7 and 14 µm.

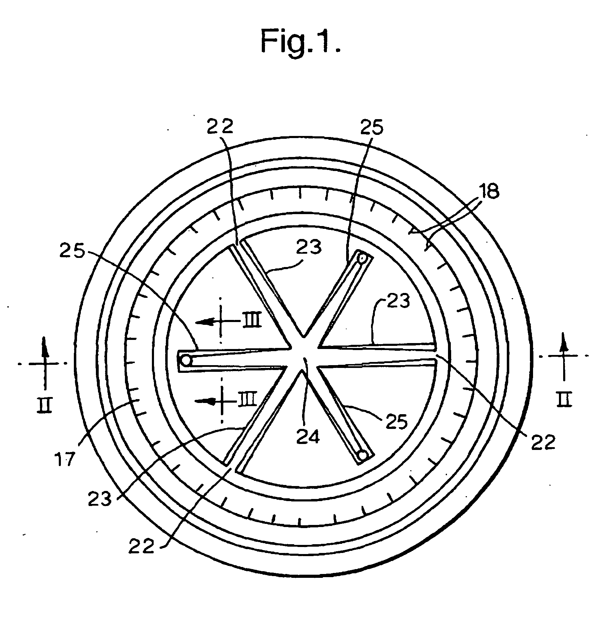

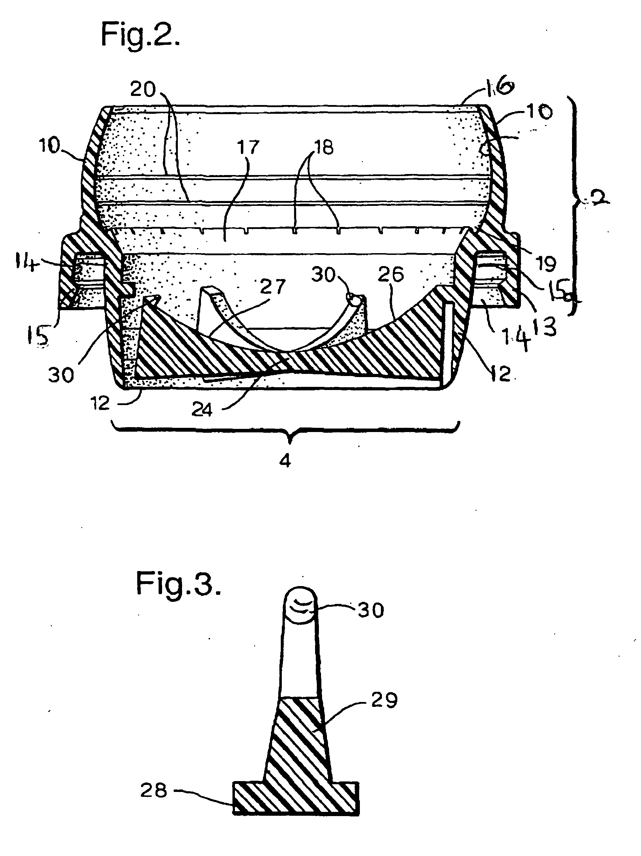

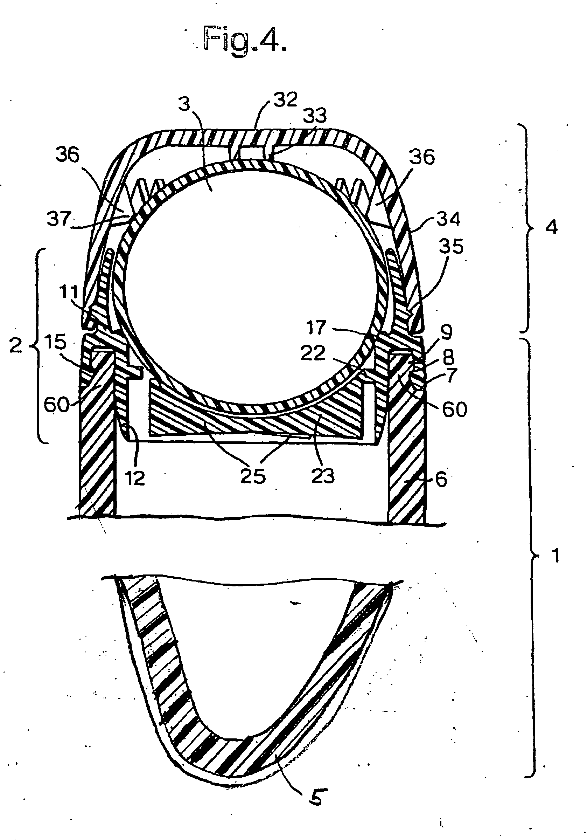

The data summarised above demonstrates that the perceived tone, radiance and/or smoothness is superior when the formulation is applied from P2, the dispenser described hereinabove in relation to Figures 1 to 4.

Comparison CA shows that in the absence of the Mica pigment, the formulation shows only minimal change of perceived tone, radiance, smoothness on application.

Comparison between CA and the Examples shows the beneficial effect of incorporating a mica pigment to cooperate with the humectant and glyceride oil in order to promote skin having a superior quality.

Examples 1 to 4 demonstrate the benefit of including a dimethicone cross polymer and alumina.

Example 6 shows that the Timiron Silk Blue is an especially effective mica pigment in terms of improving perceived tone, radiance and/or smoothness of axilla skin in the presence of the humectant and triglyceride oil.

## Claims

1. An antiperspirant roll-on emulsion comprising
an aqueous phase in which is dissolved an antiperspirant salt;
at least 1% by weight of a water-soluble aliphatic di or tri-hydric humectant or/and a glyceride oil;
at least 0.25% by weight of a fragrance oil;
at least 1.5% by weight of a nonionic emulsifier and
at least 0.25% by weight of a mica pigment.

2. A composition according to claim 1 which contains at least 2% by weight of the humectant.

3. A composition according to claim 1 or 2 in which the moisturiser is glycerol.

4. A composition according to claim 1 or 2 in which the moisturiser is polyethylene glycol having a molecular weight of from 200 to 500.

5. A composition according to any preceding claim which contains up to 8% by weight of the humectant.

6. A composition according to any preceding claim which contains at least 2% by weight of the natural glyceride oil.

7. A composition according to claim 7 which contains up to 8% by weight of the glyceride oil.

8. A composition according to any preceding claim which the glyceride oil is an ester of an unsaturated C18 acid.

9. A composition according to claim 8 in which the glyceride oil is sunflower seed oil.

10. A composition according to any preceding claim which contains both the humectant and the glyceride oil in a total amount of from 4 to 10% by weight.

11. A composition according to any preceding claim in which the mica pigment is present in an amount of from 0.4 to 2% by weight.

12. A composition according to claim 11 which contains from 0.5 to 1.2% by weight of the mica pigment.

13. A composition according to any preceding claim in which the mica pigment is present in a weight ratio to the humectant of from 2:5 to 1:10.

14. A composition according to claim 12 in which the mica pigment is present in a weight ratio to the humectant of from 1:4 to 2:15.

15. A composition according to any preceding claim in which the mica pigment is present in a weight ratio to the glyceride oil of from 2:5 to 1:10.

16. A composition according to any preceding claim in which the mica pigment has a highlight of wavelength less than 530nm.

17. A composition according to claim 16 in which the mica pigment has a highlight of wavelength less than 500nm.

18. A composition according to any preceding claim in which the mica pigment comprises a coating of titanium oxide and tin oxide.

19. A composition according to any preceding claim in which the mica pigment or mixture of mica pigments a mean particle size (D-50) of from 12 to 25 µm.

20. A composition according to any preceding claim in which the mica pigment comprises at least one pigment having a mean particle size (D-50) of below 15 µm.

21. A composition according to claim 19 or 20 in which the mica pigment or mixture of pigments has a mean particle size (D-50) of from 12 to 17 µm.

22. A composition according to claim 20 or 21 in which the mica pigment is a blue pigment, either alone or in combination with an off-white pigment with a silver lustre.

23. A composition according to any preceding claim which contains from 3 to 7.5% oils.

24. A composition according to any preceding claim which contains up to 4.5% non-ionic emulsifier.

25. A composition according to any preceding claim in which the non-ionic emulsifier is present in a weight ratio to the oils of from 1:2 to 2:1.

26. A composition according to claim 25 in which the non-ionic emulsifier is present in a weight ratio to the oils of from 3:4 to 4:3.

27. A composition according to any preceding claim which further comprises a hydrophobic silica in an amount of from 0.1 to 2% by weight.

28. A composition according to claim 27 which the hydrophobic silica is present in an amount of from 0.5 to 1% by weight.

29. A composition according to claim 27 or 28 in which the silica has an average particle size of below 500nm.

30. A composition according to any preceding claim which contains a hair growth inhibitor.

31. A composition according to claim 30 in which the hair growth inhibitor is water-soluble.

32. A composition according to any preceding claim which contains a micro-fine aluminium oxide, preferably from 0.25 to 5% by weight of the composition.

33. A composition according to any preceding claim comprising a silicone elastomer, preferably in an amount of from 0.1 to 4% by weight.

34. A composition according to claim 33 free from or containing less than 0.25 by weight of the humectant and free from or containing less than 0.25% by weight of the natural glyceride oil.

35. A method of simultaneously visually enhancing skin appearance and reducing perspiration in a localised region of skin, and particularly in the axilla, comprising topically applying to the skin an antiperspirant composition according to any of claims 1 to 34.

36. An antiperspirant product comprising an antiperspirant composition contained within a dispensing container comprising a reservoir having at one end a housing for a rotatable ball, a ball retained by the housing and having a fraction proud of the housing, and a cap that is fitted removably over the ball in which product the composition is according to any of claims 1 to 34.

37. A product according to claim 36 in which the container is incapable of standing stably in an upright orientation and the cap has a flat top which enables the container to stand stably in an invert orientation.

38. A product according to claim 36 or 37 in which the housing further comprises a spider inward of the ball that is biased into point contact with the ball and comprising a plurality of arms having a parallel surface facing the ball.

39. A product according to any of claims 36 to 38 in which the housing comprises an annular sealing ring against which the ball is pressed by fitting the cap.

40. A product according to claim 39 in which the housing further defines an annular groove outward of and contiguous with the sealing ring and the sealing ring is notched along its outward edge providing fluid communication between the reservoir and the annular groove.

41. A product according to any of claims 36 to 40 in which the housing further defines fluid flow perturbating ribs moulded outward of the sealing ring which are spaced from the ball when it is rotating.

## Patentansprüche

1. Schweißhemmende Emulsion für einen Deoroller, die folgendes aufweist:
eine wässrige Phase, in der ein schweißhemmendes Salz gelöst ist;
mindestens 1 Gew.-% eines wasserlöslichen aliphatischen 2- oder 3-wertigen Feuchthaltemittels und/oder eines Glyceridöls;
mindestens 0,25 Gew.-% eines Duftöls;
mindestens 1,5 Gew.-% eines nichtionischen Emulgators und
mindestens 0,25 Gew.-% eines Glimmerpigments.

2. Zusammensetzung nach Anspruch 1,
die mindestens 2 Gew.-% des Feuchthaltemittels enthält.

3. Zusammensetzung nach Anspruch 1 oder 2,
wobei das Feuchthaltemittel Glycerol ist.

4. Zusammensetzung nach Anspruch 1 oder 2,
wobei das Feuchthaltemittel Polyethylenglycol mit einem Molekulargewicht von 200 bis 500 ist.

5. Zusammensetzung nach einem der vorstehenden Ansprüche,
die bis zu 8 Gew.-% des Feuchthaltemittels enthält.

6. Zusammensetzung nach einem der vorstehenden Ansprüche,
die mindestens 2 Gew.-% des natürlichen Glyceridöls enthält.

7. Zusammensetzung nach Anspruch 7,
die bis zu 8 Gew.-% des Glyceridöls enthält.

8. Zusammensetzung nach einem der vorstehenden Ansprüche,
wobei das Glyceridöl ein Ester einer ungesättigten C₁₈-Säure ist.

9. Zusammensetzung nach Anspruch 8,
wobei das Glyceridöl Sonnenblumenkernöl ist.

10. Zusammensetzung nach einem der vorstehenden Ansprüche,
die sowohl das Feuchthaltemittel als auch das Glyceridöl in einer Gesamtmenge von 4 bis 10 Gew.-% enthält.

11. Zusammensetzung nach einem der vorstehenden Ansprüche,
wobei das Glimmerpigment in einer Menge von 0,4 bis 2 Gew.-% vorliegt.

12. Zusammensetzung nach Anspruch 11,
die 0,5 bis 1,2 Gew.-% des Glimmerpigments enthält.

13. Zusammensetzung nach einem der vorstehenden Ansprüche,
wobei das Glimmerpigment in einem Gewichtsverhältnis zum Feuchthaltemittel von 2:5 bis 1:10 vorliegt.

14. Zusammensetzung nach Anspruch 12,
wobei das Glimmerpigment in einem Gewichtsverhältnis zum Feuchthaltemittel von 1:4 bis 2:15 vorliegt.

15. Zusammensetzung nach einem der vorstehenden Ansprüche,
wobei das Glimmerpigment in einem Gewichtsverhältnis zum Glyceridöl von 2:5 bis 1:10 vorliegt.

16. Zusammensetzung nach einem der vorstehenden Ansprüche,
wobei das Glimmerpigment ein Maximum der Wellenlänge von weniger als 530 nm hat.

17. Zusammensetzung nach Anspruch 16,
wobei das Glimmerpigment ein Maximum der Wellenlänge von weniger als 500 nm hat.

18. Zusammensetzung nach einem der vorstehenden Ansprüche,
wobei das Glimmerpigment eine Beschichtung aus Titanoxid und Zinnoxid aufweist.

19. Zusammensetzung nach einem der vorstehenden Ansprüche,
wobei das Glimmerpigment oder das Gemisch von Glimmerpigmenten eine mittlere Partikelgröße (D-50) von 12 bis 25 µm hat.

20. Zusammensetzung nach einem der vorstehenden Ansprüche,
wobei das Glimmerpigment zumindest ein Pigment mit einer mittleren Partikelgröße (D-50) von weniger als 15 µm aufweist.

21. Zusammensetzung nach Anspruch 19 oder 20,
wobei das Glimmerpigment oder das Gemisch von Pigmenten eine mittlere Partikelgröße (D-50) von 12 bis 17 µm aufweist.

22. Zusammensetzung nach Anspruch 20 oder 21,
wobei das Glimmerpigment ein blaues Pigment, entweder allein oder in Kombination mit einem weißlichen Pigment mit einem Silberglanz, ist.

23. Zusammensetzung nach einem der vorstehenden Ansprüche,
die 3 bis 7,5 % Öle enthält.

24. Zusammensetzung nach einem der vorstehenden Ansprüche,
die bis zu 4,5 % eines nichtionischen Emulgators enthält.

25. Zusammensetzung nach einem der vorstehenden Ansprüche,
wobei der nichtionische Emulgator in einem Gewichtsverhältnis zu den Ölen von 1:2 bis 2:1 vorliegt.

26. Zusammensetzung nach Anspruch 25,
wobei der nichtionische Emulgator in einem Gewichtsverhältnis zu den Ölen von 3:4 bis 4:3 vorliegt.

27. Zusammensetzung nach einem der vorstehenden Ansprüche,
die ferner ein hydrophobes Siliciumdioxid in einer Menge von 0,1 bis 2 Gew.-% aufweist.

28. Zusammensetzung nach Anspruch 27,
wobei das hydrophobe Siliciumdioxid in einer Menge von 0,5 bis 1 Gew.-% vorliegt.

29. Zusammensetzung nach Anspruch 27 oder 28,
wobei das Siliciumdioxid eine mittlere Partikelgröße von weniger als 500 nm aufweist.

30. Zusammensetzung nach einem der vorstehenden Ansprüche,
die einen Haarwuchsinhibitor enthält.

31. Zusammensetzung nach Anspruch 30,
wobei der Haarwuchsinhibitor wasserslöslich ist.

32. Zusammensetzung nach einem der vorstehenden Ansprüche,
die ein mikrofeines Aluminiumoxid, vorzugsweise mit 0,25 bis 5 Gew.-% der Zusammensetzung, enthält.

33. Zusammensetzung nach einem der vorstehenden Ansprüche,
die ein Siliconelastomer, vorzugsweise in einer Menge von 0,1 bis 4 Gew.-%, aufweist.

34. Zusammensetzung nach Anspruch 33,
die ohne das Feuchthaltemittel ist oder weniger als 0,25 Gew.-% davon enthält und ohne das natürliche Glyceridöl ist oder weniger als 0,25 Gew.-% davon enthält.

35. Verfahren zum gleichzeitigen visuellen Verbessern des Aussehens der Haut und zum Vermindern der Schweißbildung in einem lokalisierten Bereich der Haut und insbesondere in den Achselhöhlen, das das topische Aufbringen einer schweißhemmenden Zusammensetzung nach einem der Ansprüche 1 bis 34 auf die Haut aufweist.

36. Schweißhemmendes Produkt,
das eine schweißhemmende Zusammensetzung aufweist, die in einem Spenderbehälter enthalten ist, der ein Reservoir, das an einem Ende ein Gehäuse für eine drehbare Kugel hat, eine Kugel, die vom Gehäuse gehalten wird und ein Teilstück aufweist, das über das Gehäuse übersteht, und eine Abdeckung aufweist, die abnehmbar auf der Kugel sitzt, wobei bei diesem Produkt die Zusammensetzung einem der Ansprüche 1 bis 34 entspricht.

37. Produkt nach Anspruch 36,
wobei der Behälter in aufrechter Richtung nicht stabil stehen kann und die Abdeckung eine ebene Oberseite hat, die es ermöglicht, dass der Behälter in umgekehrter Richtung stabil stehen kann.

38. Produkt nach Anspruch 36 oder 37,
wobei das Gehäuse ferner innen hinter der Kugel ein sternförmiges Teil aufweist, das in einen Kontaktpunkt mit der Kugel gedrückt wird und mehrere Arme aufweist, die eine zur Kugel zeigende parallele Oberfläche haben.

39. Produkt nach einem der Ansprüche 36 bis 38,
wobei das Gehäuse einen Dichtungsring aufweist, gegen den die Kugel gedrückt wird, wenn die Abdeckung aufgesetzt ist.

40. Produkt nach Anspruch 39,
wobei das Gehäuse ferner eine ringförmige Vertiefung außerhalb des Dichtungsrings und an diesen angrenzend bildet und der Dichtungsring entlang seines Außenrandes gekerbt ist, womit für eine Fluidverbindung zwischen dem Reservoir und der ringförmigen Vertiefung gesorgt ist.

41. Produkt nach einem der Ansprüche 36 bis 40,
wobei das Gehäuse ferner den Fluidstrom störende Rippen bildet, die außerhalb des Dichtungsrings geformt sind, die von der Kugel räumlich getrennt sind, wenn sich diese dreht.

## Revendications

1. Emulsion anti-transpirante appliquée par roulement, comprenant :
une phase aqueuse dans laquelle un sel anti-transpirant est dissous ;
au moins 1 % en poids d'un humidifiant aliphatique di- ou tri-hydrique soluble dans l'eau ou/et d'une huile de glycéride ;
au moins 0,25 % en poids d'une huile de fragrance ;
au moins 1,5 % en poids d'un émulsifiant non ionique ; et
au moins 0,25 % en poids d'un pigment de mica.

2. Composition selon la revendication 1, contenant au moins 2 % en poids de l'humidifiant.

3. Composition selon la revendication 1 ou 2, dans laquelle l'humidifiant est le glycérol.

4. Composition selon la revendication 1 ou 2, dans laquelle l'humidifiant est un polyéthylène glycol qui présente un poids moléculaire compris entre 200 et 500.

5. Composition selon l'une quelconque des revendications précédentes, contenant jusqu'à 8 % en poids de l'humidifiant.

6. Composition selon l'une quelconque des revendications précédentes, contenant au moins 2 % en poids de l'huile de glycéride naturelle.

7. Composition selon la revendication 7, contenant jusqu'à 8 % en poids de l'huiLe de glycéride.

8. Composition selon l'une quelconque des revendications précédentes, dans laquelle l'huile de glycéride est un ester d'un acide C18 insaturé.

9. Composition selon la revendication 8, dans laquelle l'huile de glycéride est l'huile de tournesol.

10. Composition selon l'une quelconque des revendications précédentes, contenant à la fois l'humidifiant et l'huile de glycéride en une quantité totale qui est comprise entre 4 % en poids et 10 % en poids.

11. Composition selon l'une quelconque des revendications précédentes, dans laquelle le pigment de mica est présent en une quantité qui est comprise entre 0,4 % en poids et 2 % en poids.

12. Composition selon la revendication 11, contenant entre 0,5 % en poids et 1,2 % en poids du pigment de mica.

13. Composition selon l'une quelconque des revendications précédentes, dans laquelle le pigment de mica est présent en un rapport de poids avec l'humidifiant qui est compris entre 2:5 et 1:10.

14. Composition selon la revendication 12, dans laquelle le pigment de mica est présent en un rapport de poids avec l'humidifiant qui est compris entre 1:4 et 2:15.

15. Composition selon l'une quelconque des revendications précédentes, dans laquelle le pigment de mica est présent en un rapport de poids avec l'huile de glycéride qui est compris entre 2:5 et 1:10.

16. Composition selon l'une quelconque des revendications précédentes, dans laquelle le pigment de mica présente une surbrillance de longueur d'onde qui est inférieure à 530 nm.

17. Composition selon la revendication 16, dans laquelle le pigment de mica présente une surbrillance de longueur d'onde qui est inférieure à 500 nm.

18. Composition selon l'une quelconque des revendications précédentes, dans laquelle le pigment de mica comprend un revêtement d'oxyde de titane et d'oxyde d'étain.

19. Composition selon l'une quelconque des revendications précédentes, dans laquelle le pigment de mica ou un mélange de pigments de mica présente une taille moyenne de particule (D-50) qui est comprise entre 12 µm et 25 µm.

20. Composition selon l'une quelconque des revendications précédentes, dans laquelle le pigment de mica comprend au moins un pigment qui présente une taille moyenne de particule (D-50) inférieure à 15 µm.

21. Composition selon l'une quelconque des revendications 19 ou 20, dans laquelle le pigment de mica ou un mélange de pigments présente une taille moyenne de particule (D-50) qui est comprise entre 12 µm et 17 µm.

22. Composition selon la revendication 20 ou 21, dans laquelle le pigment de mica est un pigment bleu, soit seul, soit en combinaison avec un pigment blanc cassé présentant un éclat d'argent.

23. Composition selon l'une quelconque des revendications précédentes, contenant entre 3 % et 7,5 % d'huiles.

24. Composition selon l'une quelconque des revendications précédentes, contenant jusqu'à 4,5 % d'un émulsifiant non ionique.

25. Composition selon l'une quelconque des revendications précédentes, dans laquelle l'émulsifiant non ionique est présent en un rapport de poids avec les huiles qui est compris entre 1:2 et 2:1.

26. Composition selon la revendication 25, dans laquelle l'émulsifiant non ionique est présent en un rapport de poids avec les huiles qui est compris entre 3:4 et 4:3.

27. Composition selon l'une quelconque des revendications précédentes, comprenant en outre une silice hydrophobe en une quantité qui est comprise entre 0,1 % en poids et 2 % en poids.

28. Composition selon la revendication 27, dans laquelle la silice hydrophobe est présente en une quantité qui est comprise entre 0,5 % en poids et 1 % en poids.

29. Composition selon la revendication 27 ou 28, dans laquelle la silice présente une taille moyenne de particule qui est inférieure à 500 nm.

30. Composition selon l'une quelconque des revendications précédentes, contenant un inhibiteur de croissance de poils.

31. Composition selon la revendication 30, dans laquelle l'inhibiteur de croissance de poils est soluble dans l'eau.

32. Composition selon l'une quelconque des revendications précédentes, contenant un oxyde d'aluminium micro-fin, qui constitue de préférence entre 0,25 % en poids et 5 % en poids de la composition.

33. Composition selon l'une quelconque des revendications précédentes, comprenant un élastomère de silicone, de préférence en une quantité qui est comprise entre 0,1 % en poids et 4 % en poids.

34. Composition selon la revendication 33, exempte ou contenant moins de 0,25 % en poids de l'humidifiant, et exempte ou contenant moins de 0,25 % en poids de l'huile de glycéride naturelle.

35. Procédé pour améliorer visuellement et simultanément l'apparence de la peau et réduire la transpiration dans une région localisée de la peau, et en particulier sous les aisselles, comprenant l'application topique sur la peau d'une composition anti-transpirante selon l'une quelconque des revendications 1 à 34.

36. Produit anti-transpirant, comprenant une composition anti-transpirante contenue à l'intérieur d'un récipient de distribution comprenant un réservoir qui présente à une extrémité un boîtier pour une bille rotative, une bille retenue par le boîtier et comprenant une protubérance fragmentaire du boîtier, et un couvercle qui est agencé de façon amovible sur la bille, produit dans lequel la composition est une composition selon l'une quelconque des revendications 1 à 34.

37. Produit selon la revendication 36, dans lequel le récipient ne peut pas être posé debout de façon stable dans une orientation verticale, et le couvercle présente un sommet plat qui permet de poser le récipient debout de façon stable dans une orientation inversée.

38. Produit selon la revendication 36 ou 37, dans lequel le boîtier comprend en outre un croisillon vers l'intérieur de la bille qui est poussé en contact ponctuel avec la bille et qui comprend une pluralité de bras qui présentent une surface parallèle en face de la bille.

39. Produit selon l'une quelconque des revendications 36 à 38, dans lequel le boîtier comprend un anneau d'étanchéité annulaire contre lequel la bille est pressée en plaçant le couvercle.

40. Produit selon la revendication 39, dans lequel le boîtier définit en outre une rainure annulaire vers l'extérieur de et contiguë à l'anneau d'étanchéité, et l'anneau d'étanchéité est encoché le long de son bord extérieur, établissant une communication fluidique entre le réservoir et la rainure annulaire.

41. Produit selon l'une quelconque des revendications 36 à 40, dans lequel le boîtier définit en outre des nervures de perturbation d'écoulement de fluide qui sont moulées vers l'extérieur de l'anneau d'étanchéité et qui sont espacées de la bille lorsque celle-ci tourne.
